# EUROPEAN PATENT SPECIFICATION

(11) **EP 4 234 715 B1**
(45) Date of publication and mention of the grant of the patent: **11.03.2026**
(21) Application number: 21890798.8
(22) Date of filing: 14.09.2021
(51) Int. Cl.: C12Q 1/6844

(54) **METHOD FOR TESTING TARGET NUCLEIC ACID IN SAMPLE**
VERFAHREN ZUM TESTEN EINER ZIELNUKLEINSÄURE IN EINER PROBE
PROCÉDÉ DE TEST D'ACIDE NUCLÉIQUE CIBLE DANS UN ÉCHANTILLON

(30) Priority: 16.11.2020 CN 202011278709
(43) Date of publication of application: 30.08.2023
(73) Proprietor: Beijing Synsortech Co., Ltd., Beijing 102600 (CN); Zheijiang Synsorbio Technology Co., Ltd., Shaoxing City, Zhejiang 312300 (CN); Beijing Synsorbio Technology Co., Ltd., Beijing 102600 (CN)
(72) Inventor: PAN, Weiye, Guangzhou, Guangdong 510405 (CN); CHEN, Chongjian, Jinhua, Zhejiang 322006 (CN); SUN, Yang, Beijing 100052 (CN); ZHANG, Zhuo, Nanyang, Henan 474350 (CN); CHENG, Xuejia, Beijing 101102 (CN); PENG, Qiongfang, Fuzhou, Jiangxi 331800 (CN)
(74) Representative: karo IP
(86) International application number: PCT/CN2021/118280
(87) International publication number: WO 2022/100270

(56) References cited:
- EP-A1- 3 029 146
- WO-A1-2020/006036
- CN-A- 109 055 499
- CN-A- 109 837 328
- CN-A- 110 184 329
- CN-A- 110 387 405
- CN-A- 110 551 800
- CN-A- 111 593 145
- CN-A- 111 630 163
- DING XIONG ET AL: "Ultrasensitive and visual detection of SARS-CoV-2 using all-in-one dual CRISPR-Cas12a assay", NATURE COMMUNICATIONS, vol. 11, no. 1, 18 September 2020 (2020-09-18), XP055810852, DOI: 10.1038/s41467-020-18575-6
- TENG, FEI ET AL.: "CDetection: CRISPR-Cas12b-based DNA detection with sub-attomolar sensitivity and single-base specificity", GENOME BIOLOGY, vol. 20, 1 July 2019 (2019-07-01), XP055817045, ISSN: 1474-760X, DOI: 10.1186/s13059-019-1742-z

## Description

### TECHNICALFIELD

The present application belongs to the technical field of gene engineering, and particularly relates to a method for detecting a target nucleic acid in a sample.

### BACKGROUND ART

In 2006, Piepenburg et al. created a new whole-process isothermal amplification technology to open double-stranded DNA by using enzymes, which is called Recombinase Polymerase Amplification (hereinafter referred to as RPA). RPA refers to the T4 bacteriophage DNA replication system. In addition to a DNA polymerase that can work at room temperature, the reaction also comprises bacteriophage recombinase, accessory protein, and DNA binding protein. RPA is represented by products of TwistDx. Recombinase-aid Isothermal Amplification (hereinafter referred to as RAA) uses the recombinase obtained from bacteria or fungi to further improve the stability of the entire amplification system.

Both RPA and RAA have the ability to rapidly complete the amplification of nearly 10 orders of magnitude dynamic range at a single temperature step close to normal temperature (37-42 °C), and has the top reaction speed among various amplification technologies. In 10 minutes, it can complete the amount of amplification that an ordinary PCR will take nearly an hour to finish. Under ideal conditions, it can also achieve sensitivity close to that of QPCR, reaching the limit of 1-20 copies/reaction. Combined with various display technologies, such as a combination of exonuclease and reporter probe, it can replace traditional PCR to achieve real-time rapid detection of various microorganisms on a fluorescent platform or a lateral flow test strip platform.

In 2018, multiple R&D teams successively published technologies such as SHERLOCK, DETECTR, and HOLMES, all of which achieve real time rapid detection of microorganisms of pathogens based on isothermal amplification in combination with CRISPR-Cas system, further improving the application range of isothermal on-site amplification and steadily increasing the reaction speed, sensitivity, specificity of isothermal amplification and dependence on fluorescence reading instruments.

RPA/RAA technology is currently known polymerase amplification system with the fastest amplification speed, but it has disadvantages of longer primer probe and stronger fault tolerance, which result in low specificity in amplification and report, and are easy to producing mismatched amplification products. The recombinase of RPA/RAA technology itself depends on the ATP fuel, which makes the nonspecific product compete heavily for the fuel resource of the specific product. As a result, the specific amplification could not produce enough products to be submitted to the reporter system until the end of the amplification, thereby affecting the detection sensitivity. Especially in clinical diagnosis, the microenvironment of a sample is usually very complicated due to the interference background of proteins and nucleic acids. Although RPA/RAA reaction has a certain tolerance to interference factors such as proteins, the instability of sensitivity and specificity caused by the interference of nucleic acid background restricts the application and promotion of RPA/RAA used alone in clinical high-sensitivity and rapid diagnosis.

WO 2020/006036 A1 provides methods and systems for amplifying and/or detecting target double-stranded or single-stranded nucleic acids. The methods comprise combining a sample comprising the target nucleic acid with an amplification reaction mixture, amplifying the target nucleic acid, and further amplifying the target nucleic acid by repeated opening, unwinding, annealing and extension under isothermal conditions. The amplification reaction mixture may include an amplification CRISPR system, a helicase, a primer pair, and a polymerase. The amplification CRISPR system may comprise a first and second CRISPR/Cas complex, the first and second CRISPR/Cas complex may comprise a first CRISPR/Cas enzyme and a first guide molecule that guides the first CRISPR/Cas complex to a first strand of the target nucleic acid; the second CRISPR/Cas complex may comprise a second CRISPR/Cas enzyme and a second guide molecule that guides the second CRISPR/Cas complex to a second strand of the target nucleic acid.

EP 3 029 146 Al provides a method for amplifying a nucleic acid molecule. The method involves mixing an RNA template with a composition having a reverse transcriptase, a DNA polymerase and a RT inhibition reducer. The RT inhibition reducer can be Sso7d, Sac7d, Sac7e, Sso7e, AluI methylase, suramin, a phosphorothioate oligodeoxycytosine, a phosphorothioate oligodeoxyadenine, a phosphorothioate oligodeoxythymine or poly(rA)(dT). The mixing forms a mixture that is incubated under conditions sufficient to synthesize a DNA molecule complementary to all or a portion of the RNA template, thereby amplifying the nucleic acid molecule.

CN 111 593 145 A discloses a one-step nucleic acid detection method using Cas12a, crRNA, and RPA components. CN 109 837 328 A highlights the superiority of Cas12b over Cas12a in RPA-amplified nucleic acid detection. CN 109 055 499 A describes a single-tube RNA detection method using Cas13b under isothermal conditions. CN 110 387 405 A presents an RAA-CRISPR system for rapid nucleic acid detection with a guide sequence for Cas13. Ding Xiong et al., "Ultrasensitive and visual detection of SARS-CoV-2 using all-in-one dual CRISPR-Cas12a assay," Nature Communications, 2020, emphasizes the need to optimize parameters like probe and primer concentrations for effective one-pot RPA-CRISPR reactions.

In combination with RPA/RAA technology, the trans-cleavage activity of Cas protein in CRISPR-Cas is applied to the downstream of RPA/RAA reaction, which can make up for insufficient signal caused by the insufficient specific amplification product mentioned above, and enable the high-sensitivity detection to be carried out in a more extensive environment with no need for too precise optical instrument and equipment. The Cas12b system-based CDetection system discovered and reported by Li Wei et al. has good tolerances for buffer system and temperature, and has been proven to have certain detection sensitivity in single tube step-by-step reactions. However, the issue of mutual interference between CDetection and RPA/RAA has not been resolved, and the reaction still needs to be carried out by two steps of isothermal amplification and CDetection, which cannot achieve synchronous reaction. There is no detailed external report on the mechanism leading to the difficulty, and the possibilities according to the reaction mechanism are as follows.
a. The composition of enzymes in RPA/RAA is relatively complex, and cannot tolerate large changes in ion concentration, and thus the concentrations of the main salts and additives in the buffer system depend on the design of the amplification system itself, and these factors lead to changes in the conformation of sgRNA, thereby further affecting the effectiveness of the sgRNA-Cas complex.
b. If the amount of the Cas12-sgRNA system is simply increased to make up for the above defects, the template will be cut off by the Cas protein during the RPA/RAA amplification process, and thus cannot enter the subsequent exponential amplification, thereby affecting the amplification linearity and sensitivity of the reaction. In addition, the trans-cleavage activity of the Cas12-sgRNA system will continuously cleave and consume the primer required for the amplification reaction, thereby directly affecting the amplification upper limits of RPA and RAA.
c. RPA/RAA comprises a large number of single and double stranded binding proteins, all of which compete with Cas proteins for substrates. In the system dominated by the former, Cas cis/trans cleavage cannot proceed smoothly. After diluting the products of RPA/RAA reaction, the ratio of single and double stranded binding proteins to the Cas protein is reduced, thereby recovering the dominance of the Cas-sgRNA system reaction to quickly generate the cleavage signal.

### SUMMARY

For addressing the shortcoming in the existing technology, the present application provides a method for detecting a target nucleic acid in a sample.

Particularly, the present application relates to the following aspects:

In one aspect, the present invention relates to a method for detecting a target nucleic acid in a sample, comprising the following steps:
reacting the sample with a mixed reaction system consisting of a sgRNA-Cas system and a recombinase-aid isothermal amplification system; and
detecting a detectable signal generated by the reaction after the reaction is completed;
wherein the sgRNA-Cas system comprises a Cas12b protein and a sgRNA targeting the target nucleic acid; and the recombinase-aid isothermal amplification system comprises a primer and a single-stranded DNA reporter molecule that generates the detectable signal after being cleaved; wherein the system further comprises suramin and
tRNA, and wherein the target nucleic acid is RNA.

In another aspect, the present invention relates to a method as above-mentioned, wherein a concentration of the sgRNA in the mixed reaction system is 2-80ng/µl_{∘}

The method as above-mentioned, wherein a concentration of the primer in the mixed reaction system is 300-1200nM.

The method as above-mentioned, wherein a concentration of the single-stranded DNA reporter molecule is 200-2000nM, preferably 700-1500nM.

The method as above-mentioned, wherein the reaction is performed at a first temperature and is inactivated at a second temperature, and the second temperature is greater than the first temperature.

The method as above-mentioned, wherein the second temperature is greater than 47°C.

The method as above-mentioned, wherein the mixed reaction system further comprises a divalent cation, and a concentration of the divalent cation is 8mM-25mM.

The method as above-mentioned, wherein the divalent cation is one or more selected from the group consisting of: magnesium ion, calcium ion, manganese ion, cesium ion, nickel ion, iron ion, and cobalt ion.

The method as above-mentioned, wherein a concentration of the suramin in the mixed reaction system is 3-30ng/µl, preferably 3-12ng/µl.

The method as above-mentioned, wherein a concentration of the tRNA in the mixed reaction system is 10-20ng/µl.

The method as above-mentioned, wherein the sample is selected from the group consisting of: whole blood, plasma, serum, cerebrospinal fluid, urine, feces, oral swab, nasopharyngeal swab, saliva, cell or tissue extract.

By ameliorating the mutual inhibition between recombinant polymerase amplification and sgRNA-Cas reaction, the present application can achieve that rapid and effective signal report of recombinant polymerase amplification and CDetection reaction with only one sample adding and mixing operation and a simple change of temperature, leaving out a sample adding and mixing operation of stopping the reaction midway to add components of the second step CDetection reaction. This greatly improves the convenience of operation, and reduces the aerosol pollution of recombinant polymerase amplification products generated by opening the reaction tube midway. Particularly, there are the following advantages.
1. The operation steps for a reagent user can be reduced, and uniformity and coherence of the operation are better, thereby facilitating to improve the precision of the reaction.
2. The operation of opening the reaction tube after amplification reaction of the recombinant polymerase is omitted. In addition, when these two reactions are mixed, the cis-cleavage activity of Cas protease will digest the amplification product, so that it will not be released into the environment to become a pollution source, which is especially important for high-throughput and high sensitivity detection. Aerosol contamination of the reaction product is often a headache in nucleic acid detection, and is also a fundamental reason why PCR laboratories need to improve their requirements on environmental zoning. Since the pollution source no longer exists, and the environmental requirements for the testing laboratories have been greatly reduced, it is no need to perform sample addition and amplification detection reaction by zoning.
3. By adding two additives, the sensitivity of RNA template detection is increased from less than 1E5 to 1E1 per reaction, with an increase ratio of over 10,000 times.

### BRIEF DESCRIPTION OF THE DRAWINGS

Fig.1 shows the effect of sgRNA dosage on the intensity of positive signal in a mixed reaction system.
Fig.2 shows the effect of primer dosage on the intensity of positive signal in a mixed reaction system.
Fig.3 shows the effects of magnesium ion concentration and reaction temperature on the intensity of positive signal in a mixed reaction system.
Fig.4 shows the effect of reaction time on the intensity of positive signal in a mixed reaction system.

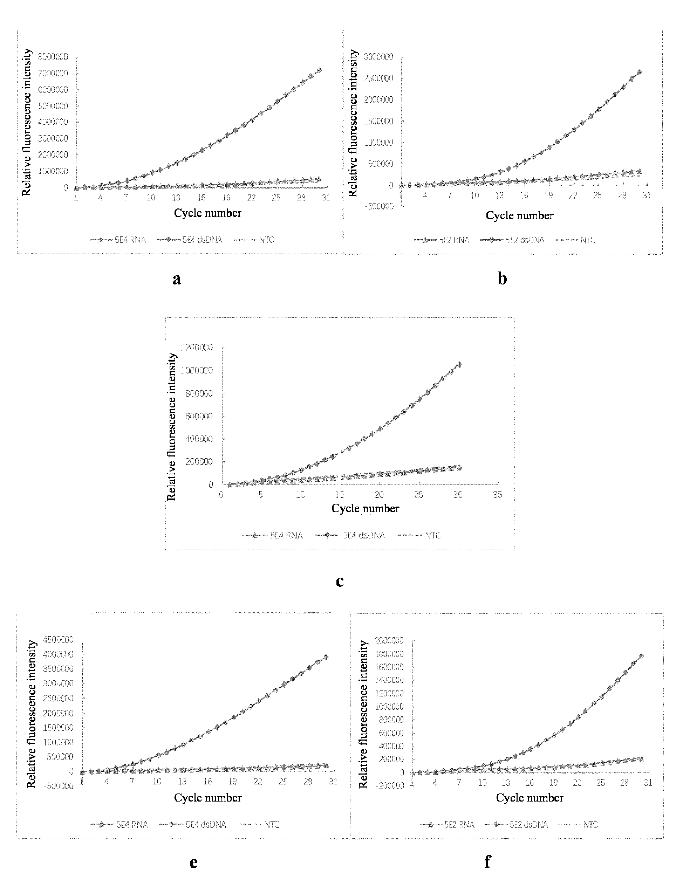

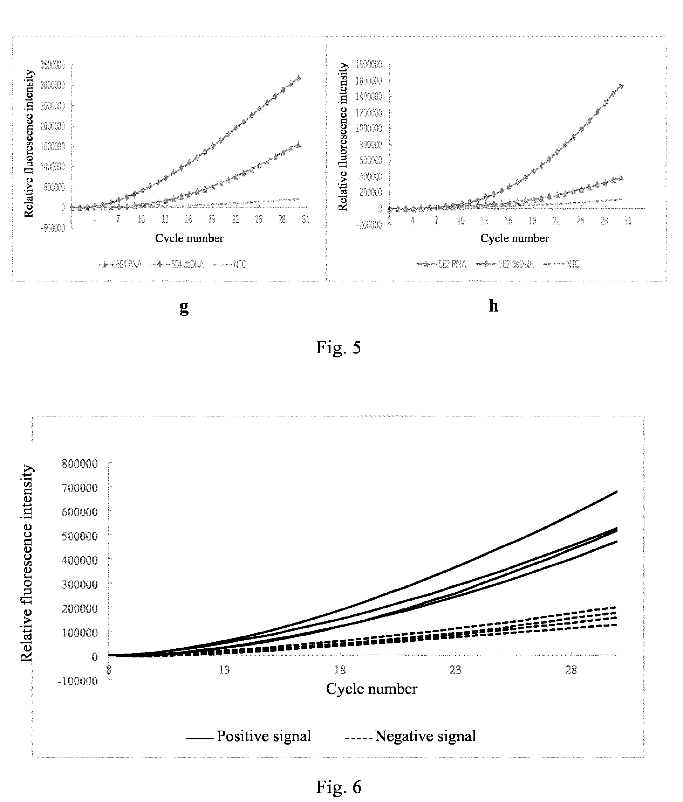

Fig.5 shows the effect of the additive on the separation degree of negative and positive in a mixed reaction system, in which: a shows the separation degree of negative and positive with respective addition of 5E4 copies of RNA per reaction, 5E4 copies of double-stranded DNA per reaction, and the same volume of water as the sample, in the case that no additive is added; b shows the separation degree of negative and positive with respective addition of 5E2 copies of RNA per reaction, 5E2 copies of double-stranded DNA per reaction, and the same volume of water as the sample, in the case that no additive is added; c shows the separation degree of negative and positive with respective addition of 5E4 copies of RNA per reaction, 5E4 copies of double-stranded DNA per reaction, and the same volume of water as the sample, in the case that 200ng suramin is added; e shows the separation degree of negative and positive with respective addition of 5E4 copies of RNA per reaction, 5E4 copies of double-stranded DNA per reaction, and the same volume of water as the sample, in the case that 500ng tRNA is added; f shows the separation degree of negative and positive with respective addition of 5E2 copies of RNA per reaction, 5E2 copies of double-stranded DNA per reaction, and the same volume of water as the sample, in the case that 500ng tRNA is added; g shows the separation degree of negative and positive with respective addition of 5E4 copies of RNA per reaction, 5E4 copies of double-stranded DNA per reaction, and the same volume of water as the sample, in the case that 200ng suramin and 500ng tRNA are added; h shows the separation degree of negative and positive with respective addition of 5E2 copies of RNA per reaction, 5E2 copies of double-stranded DNA per reaction, and the same volume of water as the sample, in the case that 5200ng suramin and 500ng tRNA are added.
Fig.6 shows the result of virus infection detected by the method according to the present application in Test Example 1.
Fig.7 shows the effect of different probe concentrations on the intensity of negative and positive signals in a mixed reaction system.
Fig. 8 shows the result of virus infection detected by the method according to the present application in Test Example 2.

### DETAILED DESCRIPTION

The present application will be further described below in conjunction with examples, and it will be understood that the examples are only used to further describe and explain the present application, and are not intended to limit the present application.

Unless defined otherwise, the technical and scientific terms in the specification have the same meaning as commonly understood by those skilled in the art. Although similar or identical methods and materials described herein may be applied in the experiments or in the practical applications, the materials and methods are described below. In the case of a conflict, the present description, including the definitions therein, shall prevail. In addition, the materials, methods and examples are provided for illustrative purposes only and are not limiting. The present application is further described below in conjunction with specific examples, but not to limit the scope of the present application.

The present application provides a method for detecting a target nucleic acid in a sample.

The method comprises the following steps:
reacting the sample with a mixed reaction system consisting of a sgRNA-Cas system and a recombinase-aid isothermal amplification system;
detecting a detectable signal generated by the reaction after the reaction is completed;
wherein the sgRNA-Cas system comprises a Cas12b protein and a sgRNA targeting the target nucleic acid; and the recombinase-aid isothermal amplification system comprises a primer and a single-stranded DNA reporter molecule that generates the detectable signal after being cleaved, wherein the system further comprises suramin and
tRNA, and wherein the target nucleic acid is RNA.

Particularly, the sgRNA (small guide RNA), i.e., single molecule guide RNA, is an important component of the CRISPR gene knockout and knockin system. Single molecule guide RNA acts in a post transcriptional modification process called RNA editing in the kinetoplastid. It is also a small non-coding RNA which can pair with pre-mRNA, and some uracil (U) may be inserted into it to produce functional mRNA. The RNA molecule edited by the guide RNA is approximately 60-80 nucleotides in length, it is transcribed by a single gene and has a tail of 3' oligomeric U, a middle sequence fragment which is precisely complementary with the edited mRNA, and a 5' end anchoring sequence which is complementary with the unedited mRNA sequence.

The Cas12b protein refers to a sequence-specific nuclease directed by RNA from microbial CRISPR system. Cas12b can target and cleave DNA target sequences under the direction of guide RNA to form DNA double-strand breaks, also known as classical dsDNA cleavage activity. More importantly, after recognizing and binding to the corresponding target DNA sequence, the complex of Cas 12b and gRNA can activate its non-specific single-stranded DNA cleavage activity, also known as non-classical bypass ssDNA cleavage activity. Utilizing non-classical bypass ssDNA cleavage activity, a single stranded DNA reporter molecule is cleaved to generate a detectable signal, which can reflect the presence and/or amount of target DNA.

The Cas12b protein may be AaCas12b protein derived from *Alicyclobacillus acidiphilus,* AkCas12b protein derived from *Alicyclobacillus kakegawensis,* AmCas12b protein derived from *Alicyclobacillus macrosporangiidus,* BhCas12b protein derived from *Bacillus hisashii,* BsCas12b protein derived from the genus *Bacillus,* Bs3Cas12b protein derived from the genus *Bacillus,* DiCas12b protein derived from *Desulfovibrio inopinatus,* LsCas12b protein derived from *Laceyell asediminis,* SbCas12b protein derived from *Spirochaetes bacterium,* or TcCas12b protein derived from *Tuberibacillus calidus.*

The "single-strand DNA reporter molecule which produces a detectable signal after being cleaved" is used to indirectly reflect the amount of target nucleic acid contained in the sample. The single-stranded DNA reporter molecule may comprise a fluorophore and a quenching group thereof at both ends of the single-stranded DNA, respectively. When the single-strand DNA is not cleaved, the fluorophore does not fluoresce due to the presence of the quenching group. When the Cas12b-gRNA complex is activated by the target nucleic acid molecule, and the DNA single strand of the single-strand DNA reporter molecule is cleaved by its non-canonical bypass ssDNA cleavage activity, the fluorophore is released and thus fluoresce. The suitable fluorophore and its corresponding quenching group, as well as the method for labeling nucleic acid molecules thereof, are known in the art. The suitable fluorophore comprises, but is not limited to: FAM, TEX, HEX, Cy3, or Cy5. The suitable quenching group comprises, but is not limited to: BHQ1, BHQ2, BHQ3, or TAMRA. The suitable fluorophore-quenching group pair comprises, but is not limited to: FAM-BHQ1, TEX-BHQ2, Cy5-BHQ3, Cy3-BHQ1, or FAM-TAMRA. Therefore, in some embodiments, the detectable signal is a fluorescent signal. In some embodiments, the fluorophore is FAM, and the quenching group is BHQ1.

The length of the single-stranded DNA in the single-stranded DNA reporter molecule may be about 2-100 nucleotides, for example, 2-5, 2-10, 2-15, 2-20, 2-25, 2-30, 2-40, or 2 to more nucleotides. The single-stranded DNA in the single-stranded DNA reporter molecule may comprise any sequence. In some embodiments, the single-stranded DNA in the single-stranded DNA reporter molecule may be selected from polyA (polyadenylic acid), polyC (polycytidylic acid), or polyT (polythymidylic acid).

Recombinase Polymerase Amplification (RPA) is known as a nucleic acid detection technology that can replace PCR. RPA technology relies primarily on three enzymes: recombinases that can bind single-stranded nucleic acids (oligonucleotide primers), single-stranded DNA binding proteins (SSBs), and strand displacement DNA polymerases.

The protein-DNA complex formed by the combination of the recombinase and the primer can search for homologous sequences in double-stranded DNA. Once the primer locates the homologous sequence, a strand exchange reaction takes place to initiate DNA synthesis to amplify the target region on the template by strand displacement. The displaced DNA strand binds to SSB to form a stable single-stranded structure, waiting for a new primer synthesis reaction to proceed. In this system, primers anchored on complementary strands with 3' tail to tail opposition are co-initialized to form a double-stranded amplicon, and this circulation goes on continuously. The whole process is carried out very fast, and generally a detectable level of amplification products can be obtained within ten minutes.

Conventional PCR must go through three steps of denaturation, annealing and extension, and the PCR machine is essentially a device that controls the rise and fall of temperature. The RPA reaction has the optimum temperature of 37-42°C, and can be carried out at room temperature without denaturation. This undoubtedly greatly speeds up PCR. In addition, since no temperature control equipment is required, RPA can truly realize portable rapid nucleic acid detection.

The RPA detection is of very high sensitivity, and it can amplify a trace amount of nucleic acid (especially DNA) template to a detectable level, and an amplification product with a length of about 1012 can be obtained from a single template molecule. Moreover, RPA does not require complicated sample processing, and is suitable for field detection where nucleic acid cannot be extracted.

RPA can amplify both DNA and RNA, thus it is no need for additional cDNA synthesis step. Not only endpoint detection can be performed on amplification products, but also an amplification process can be monitored in real time, and even the results can be read with test strips (lateral flow chromatography test strips LFD).

Similar to RPA, Recombinase-aid Isothermal Amplification (hereinafter referred to as RAA) uses the recombinase obtained from bacteria or fungi to further improve the stability of the entire amplification system. As described in the "background art" section, in the prior art some people have tried to combine the recombinase-aid isothermal amplification system and the sgRNA-Cas system for nucleic acid detection. However, it is essentially a two-step, two-pot reaction, firstly the nucleic acid in the sample is amplified by the recombinase-aid isothermal amplification system, and then the sgRNA-Cas system is used to detect the amount of nucleic acid. In view of various problems existing in the prior art, by forming a mixed reaction system with the sgRNA-Cas system and the recombinase-aid isothermal amplification system, and optimizing the reaction conditions of the mixed reaction system, the present application realizes one-step detection of the nucleic acid in a sample.

In the above mixed reaction system, the contents of the sgRNA, primer, and single-stranded DNA reporter molecule can be adjusted according to actual needs.

In a particular embodiment, the concentration of the sgRNA in the mixed reaction system is 2-80ng/µl; for example, 2-80ng/µl, 10-70ng/µl, 20-60ng/µl, 30-50ng/µl, 2ng/µl, 4ng/µl, 10ng/µl, 20ng/µl, 30ng/µl, 40ng/µl, 50ng/µl, 60ng/µl, 70ng/µl, or 80ng/µl.

In a particular embodiment, the concentration of the primer in the mixed system is 300-1200nM; for example, 300-1200nM, 400-1100nM, 500-1000nM, 600-900nM, 700-800nM, 300nM, 400nM, 500nM, 600nM, 700nM, 800nM, 900nM, 1000nM, 1100nM, or 1200nM.

In a particular embodiment, the concentration of the single-stranded DNA reporter molecule is 200-2000nM, preferably 700-1500nM. For example, it may be 200-2000nM, 300-1900nM, 400-1800nM, 500-1700nM, 600-1600n, 700-1500nM, 800-1400nM, 900-1300nM, 1000-1200nM, 200-700nM, 1500-2000nM, 200nM , 400nM, 500nM, 600nM, 700nM, 800nM, 900nM, 1000nM, 1100nM, 1200nM, 1300nM, 1400nM, 1500nM, 1600nM, 1700nM, 1800nM, 1900nM, or 2000nM.

In the present application, the one-step reaction is realized by precisely controlling the concentration of the single-stranded DNA reporter molecule in the reaction system. By in-depth research, the inventors of the present application found that the reaction time is subject to the intensity of signal generated. By increasing the concentration of the substrate (i.e., single-stranded DNA reporter molecule) in the trans-cleavage reaction, the intensity of the generated fluorescence can reach the detection sensitivity of the instrument in a shorter time, and sufficient signals can also be generated when the number of target DNA fragments in the system is low.

Further, the reaction is performed at a first temperature, and is inactivated at a second temperature, wherein the second temperature is higher than the first temperature, i.e., the temperature used for inactivation is higher than the temperature during the reaction. In a particular embodiment, the second temperature is greater than 47°C, for example, it may be 48°C, 50°C, 55°C, 60°C, 65°C, 70°C, 75°C, 80°C, or 85°C.

Further, the mixed reaction system further comprises a divalent cation, and the concentration of the divalent cation is 8-25mM; for example, may be 8-25mM, 10-20mM, 12-18mM, 8mM, 9mM, 10mM, 11mM, 12mM, 13mM, 14mM, 15mM, 16mM, 17mM, 18mM, 19mM, 20mM, 21mM, 22mM, 23mM, 24mM, or 25mM.

The divalent cation is one or more selected from the group consisting of: magnesium ion, calcium ion, manganese ion, cesium ion, nickel ion, iron ion, and cobalt ion.

The target nucleic acid in the present application is RNA, the mixed reaction system further comprises both suramin and tRNA.

When the mixed reaction system comprises suramin, the concentration of the suramin in the mixed reaction system is 3-30 ng/µl, preferably 3-12 ng/µl. For example, it may be 3-30ng/µl, 3-12ng/µl, 12-30ng/µl, 5-25ng/µl, 10-20ng/µl, 3ng/µl, 5ng/µl, 8ng/µl, 10ng/µl, 12ng/µl, 15ng/µl, 18ng/µl, 20ng/µl, 22ng/µl, 25ng/µl, 28ng/µl, or 30ng/µl.

In the present application, the one-step reaction is realized by precisely controlling the concentration of suramin in the reaction system. Through in-depth research, the inventors of the present application found that suramin is a repressor of the hydrolysis center of the triphosphate bonds of some enzymes, and polymerase, RT enzyme, and recombinase etc. comprised in the recombinase-aid amplification system (RT-RAA) rely on the hydrolysis of triphosphate bonds to generate activity. By adjusting the concentration of the suramin, the order of various enzyme reactions in the reaction system can be coordinated. In the present application, 3-30 ng/µl suramin is used to inhibit the overactive DNA polymerase and recombinase, so that the RT enzyme can combine with a RNA template-primer complex earlier to generate cDNA template, and thus the whole tandem reaction system can proceed smoothly.

When the mixed reaction system comprises tRNA, the concentration of the tRNA in the mixed reaction system is 10-20ng/µl. For example, it may be 10-20ng/µl, 12-18ng/µl, 10ng/µl, 11ng/µl, 12ng/µl, 13ng/µl, 14ng/µl, 15ng/µl, 16ng/µl, 17ng/µl, 18ng/µl, 19ng/µl, or 20ng/µl.

In the present application, the one-step reaction is realized by precisely controlling the concentration of tRNA in the reaction system. In many reports, the addition of a certain amount of background RNA can improve the sensitivity of RT-PCR, which mainly is due to the fact that improvement of the structural diversity of template RNA helps to unravel part of the higher-order structures and enable trace templates to reduce the nonspecific adsorption to other substances, thereby further facilitating its amplification reaction. Also in RT-RAA, the inventors of the present application found that the addition of the additive can also have a relatively significant effect in the case of insufficient RT enzyme activity.

To sum up, by regulating the concentration of the sgRNA in the reaction system to be 2-80 ng/µl, the concentration of the primer to be 300-1200nM, the concentration of the single-stranded DNA reporter molecule to be 200-2000nM and the likes, the present application can improve the mutual inhibition between recombinant polymerase amplification and the sgRNA-Cas reaction, the recombinant polymerase amplification and CDetection reaction may achieve fast and effective signal reporting only by one sample addition and mixing and simple change of temperature, thereby greatly improving the convenience of operation and reducing the pollution.

The sample described in the present application may be selected from the group consisting of: whole blood, plasma, serum, cerebrospinal fluid, urine, feces, buccal swab, nasopharyngeal swab, saliva, cell or tissue extract.

### EXAMPLES

### Example 1: Effect of sgRNA dosage on the reaction

### 1. Pre-preparation of a sgRNA-Cas pre-preparation system (C mixture)

The sgRNA-Cas pre-preparation system was prepared according to the dosage of each component in Table 1 below, then placing at room temperature for use after the preparation was completed.

**Table 1. Contents and addition order of components in the sgRNA-Cas pre-preparation system**

| Component | Addition amount(µl) |
|---|---|
| AaCas12b protein (0.4mg/ml)⁴ | 0.6 |
| RNA nuclease inhibitor (40U/µl)³ | 1.2 |
| Single molecule guide RNA (Xng/µl)¹ | 1 |
| Reporter probe (100µM)² | 1 |
| Nuclease-free water | 6 |
| Total (µl) | 9.8 |

| | |
|---|---|
| ¹ The sequence of the single molecule guide RNA is: 5‘-GUCUAAAGGACAGAAUUUUUCAACGGGUGUGCCAAUGGCCACUUUCCAGGU GGCAAAGCCCGUUGAACUUCAAGCGAAGUGGCACACUCAAUACUUGAGCACAC U-3’(SEQ IDNO:1). ² The sequence of the reporter probe is: 5'-FAM-TTTTTTT-BHQ1-3', in which the reporter probe is the single-stranded DNA reporter molecule described in the present application. ³ The RNA nuclease inhibitor was purchased from promega, with the catalog number N2515. ⁴ The AaCas12b protein was purchased from Nanjing Vazyme Biotech Co., Ltd. with the catalog number EN311-PE. | |

The information about each component in Table 1 is also applicable to the following tables, unless otherwise stated.

The concentration of the single molecule guide RNA in this example is 20ng/µl, 80ng/µl and 240ng/µl, respectively, in which 1µl of the nuclease-free water was used to replace the single molecule guide RNA.

### 2. Preparation of a recombinase-aid isothermal amplification system (A mixture)

The recombinase-aid amplification system was prepared according to the dosage of each component in Table 2 below, then placing at room temperature for use after the preparation was completed.

**Table 2. Contents and addition order of components in the recombinase-aid isothermal amplification system**

| Component | Addition amount (µl) |
|---|---|
| Nuclease-free water | 29.6 |
| Amplification buffer A^{l} | 12.5 |
| Magnesium acetate solution (280mM)² | 2.5 |
| The mixed solution of RAA amplification forward and reverse primers | 0.4 |
| Total (µl) | 45 |

| | |
|---|---|
| ¹ Amplification buffer A is 20% PEG35K purchased from sigma-aldrich with catalog number 818892, and was diluted to 20% for use. ² Magnesium acetate solution was purchased from sigma-aldrich with the catalog number 63052, and was diluted to 280mM for use. ³ The sequence of the forward primer is: 5'-GTTGTAGCTTGTCACACCGTTTCTATAGATTAGC-3' (SEQ ID NO:2). | |

The sequence of the reverse primer is: 5'-CCTGGTTTAACATATAGTGAACCGCCACACAT-3' (SEQ ID NO:3).

### 3. Mix of the systems

1 µl of viral nucleic acid sample (2019-nCoV RdRP pseudovirus, the nucleic acid was extracted with Magen nucleic acid extraction kit, IVD5412, and after the extraction it was diluted to 500 copies per microliter for use) was added into 45µl of A mixture, followed by adding 9.8µl of C mixture. Particularly, the 2019-nCoV RdRP gene pseudovirus was purchased from Xiamen Zeesan Biotechnology Co., Ltd with the catalog number JBD249.

The above mixture was added to the enzyme dry powder (RT-RAA dry powder, purchased from Hangzhou ZC Bio-Sci&Tech Co., Ltd., with the catalog number S0040ZC) to incubate at 42°C for 1min × 60 cycles, collecting fluorescence once per minute.

In this method, the FAM group was used as the reporter group, and the presence or absence of the target nucleic acid was judged according to the relative fluorescence signal intensity of the FAM group at 30 min.

The addition amount of the sgRNA is 0ng, 20ng sgRNA, 80ng sgRNA, and 240ng sgRNA, respectively, and the corresponding final molar concentration in the mixed reaction system is 0, 5.84nM, 23.36nM, and 70.08nM, respectively.

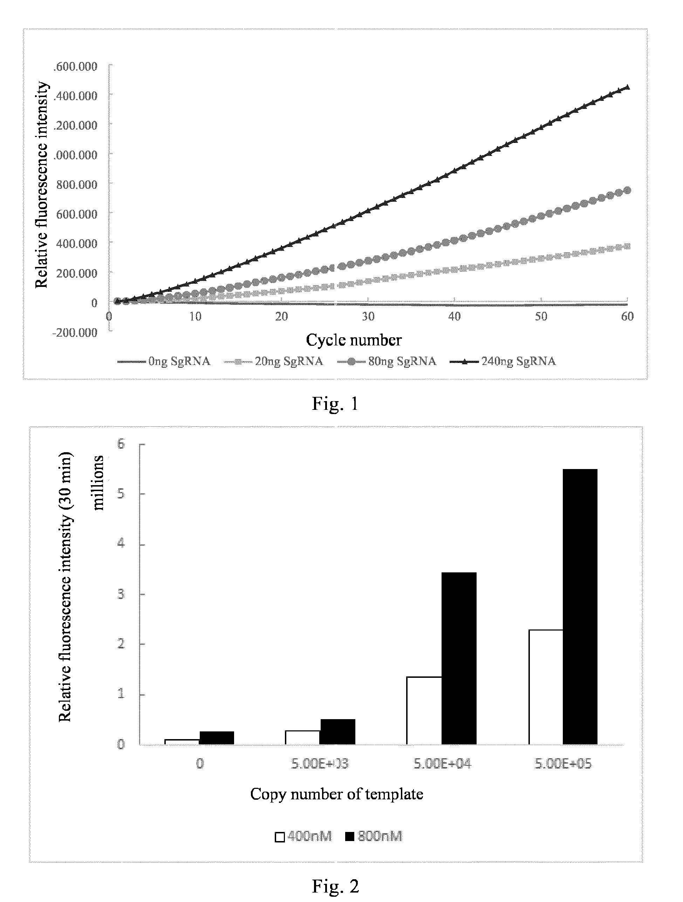

The obtained detection result is shown in Fig. 1.

It can be seen from Fig. 1 that, the increase of the sgRNA dosage increases the number of effective sgRNA-Cas complexes, and increases the number of cleaved probes, so that more FAM fluorophores are released away from the quenching group, thereby increasing the intensity of the fluorescence signal of the positive reaction and widening the distance from the intensity of the negative signal. Therefore, the sensitivity of the entire system is improved, and a lower number of viruses can be detected within a limited reaction time, so that infected persons with low virus content in the body are free from false-negative detection result.

Due to the complex internal structure of the sgRNA, some wrong structures may occur during folding, so that part of the added sgRNA becomes invalid sgRNA that cannot form a complex with the AaCas12b protein. In this application, the addition amount of the sgRNA is increased to make up for the invalid sgRNA in the system, thereby increasing the number of sgRNA-AaCas12b complexes to increase the signal intensity within a specific reaction time.

### Example 2: Effect of primer dosage on the reaction (C mixture)

### 1. Pre-preparation of a sgRNA-Cas pre-preparation system

The sgRNA-Cas pre-preparation system was prepared according to the dosage of each component in Table 3 below, then placing at room temperature for use after the preparation is complete.

**Table 3. Contents and addition order of components in the sgRNA-Cas pre-preparation system**

| Component | Addition amount (µl) |
|---|---|
| AaCas12b protein (0.4mg/ml) | 0.5 |
| RNA nuclease inhibitor (40U/µl) | 1.3 |
| Single molecule guide RNA (200ng/µl) | 1.2 |
| Nuclease-free water | 1 |
| Reporter probe (100µM) | 1 |
| Total (µl) | 5 |

### 2. Preparation of a recombinase-aid isothermal amplification system (A mixture)

The recombinase-aid isothermal amplification system was prepared according to the dosage of each component in Table 4 below, then placing at room temperature for use after the preparation was completed.

**Table 4. Contents and addition order of components in the recombinase-aid isothermal amplification system**

| Component | Addition amount (µl) |
|---|---|
| Nuclease-free water | 29.6 |
| Amplification buffer A | 12.5 |
| Magnesium acetate solution (280mM) | 2.5 |
| The mixed solution of RAA amplification forward and | 0.4 |
| reverse primers (50µM) | |
| Total (µl) | 45 |

### 3. Mix of the systems

45µl of A mixture and 1µl of viral nucleic acid sample (2019-nCoV RdRP pseudovirus, the nucleic acid was extracted with Magen nucleic acid extraction kit, IVD5412, and after the extraction it was diluted to 50,000 copies per microliter and 5,000 copies permicroliter for use), and 5µl of C mixture were added into an empty tube. Particularly, the 2019-nCoV RdRP gene pseudovirus was purchased from Xiamen Zeesan Biotechnology Co., Ltd. with the catalog number JBD249.

51µl of the above system was added into the enzyme dry powder (RT-RAA dry powder, purchased from Hangzhou ZC Bio-Sci&Tech Co., Ltd. with catalog number S0040ZC), then shaking for 8-10 seconds to mix well.

The above system was placed in a real-time fluorescence quantitative PCR instrument, the incubation program was: 45 °C, 1 min × 60 cycles, collecting fluorescence once per minute. In this method, the FAM group was used as the reporter group, and the presence or absence of the target nucleic acid was judged according to its relative fluorescence signal intensity at 60 min.

The obtained detection result is shown in Fig. 2.

As shown in Fig. 2, when the final concentration of the primer in the system was increased from 400nM to 800nM, the separation degree of the positive and negative was respectively increased by 37% (the separation degree was increased from 3.43 to 4.70) and 32% (the separation degree was increased from 5.42 to 7.17) after reaction for 30min with sample amounts of 50,000 copies and 5,000 copies. It demonstrates sidely that increase of the concentration of the primer can improve the amplification efficiency of the isothermal amplification reaction, increase the content of specific amplification product in the system, and thus improve the positive signal of the positive sample under the premise of stable negative, thereby facilitating to distinguish a negative sample from a positive sample to improve the positive and negative detection rates in the actual application process.

### Example 3: Effect of reaction temperature on the reaction result

### 1. Pre-preparation of a sgRNA-Cas pre-preparation system (C mixture)

The sgRNA-Cas pre-preparation system was prepared according to the dosage of each component in Table 5 below, then placing on ice for use after the preparation was completed.

**Table 5. Contents and addition order of components in the sgRNA-Cas pre-preparation system**

| Component | Addition amount (µl) |
|---|---|
| AaCas12b protein (0.4mg/ml) | 0.5 |
| RNA nuclease inhibitor (40U/µl) | 1.2 |
| Single molecule guide RNA (Xng/µl) | 1 |
| Nuclease-free water | 2.3 |
| Total (µl) | 5 |

The concentration X of the single molecule guide RNA is 10ng/µl, 50ng/µl, 100ng/µl and 200ng/µl, respectively.

### 2. Preparation of a recombinase-aid isothermal amplification system (A mixture)

The recombinase-aid isothermal amplification system was prepared according to the dosage of each component in Table 6 below, then placing at room temperature for use after the preparation was completed.

**Table 6. Contents and addition order of components in the recombinase-aid isothermal amplification system**

| Component | Addition amount (µl) |
|---|---|
| Nuclease-free water | 28.825-X |
| Amplification buffer A | 12.5 |
| Magnesium acetate solution (280mM) | X |
| Transfer RNA (1000ng/µl) | 0.5 |
| Suramin (800ng/µl) | 0.375 |
| The mixed solution of RAA amplification forward and reverse primers | 0.8 |
| Reporter probe | 1 |
| Total (µl) | 44 |

The addition amount of magnesium acetate solution in the above system is 1.429 µl and 2.5 µl, respectively.

Particularly, the sequence of the single-molecule guide RNA, the sequence of the reporter probe, and the sequences of the forward and reverse primers are the same as those in Example 1. The sources of components such as the amplification buffer A, magnesium acetate solution, the RNA nuclease inhibitor, and the AaCas12b protein are the same as those in Example 1, and the transfer RNA was purchased from Invitrogen with the catalog number AM7119.

### 3. Mix of the systems

44µl of A mixture, 1µl of viral nucleic acid sample (2019-nCoV RdRP pseudovirus, the nucleic acid was extracted with Magen nucleic acid extraction kit, IVD5412, and after the extraction it was diluted to 100 copies per microliter for use), and 5µl of C mixture were added into an empty tube. Particularly, the 2019-nCoV RdRP gene pseudovirus was purchased from Xiamen Zeesan Biotechnology Co., Ltd. with the catalog number JBD249.

50 µl of the above system was added into the enzyme dry powder (RT-RAA dry powder, purchased from Hangzhou ZC Bio-Sci&Tech Co., Ltd. with catalog number S0040ZC), then shaking for 8-10 seconds to mix well.

The above system was placed in a real-time fluorescence quantitative PCR instrument, the incubation program was: 45 °C or 42 °C, 1 min × 60 cycles, collecting fluorescence once per minute. In this method, the FAM group was used as the reporter group, and the presence or absence of the target nucleic acid was judged according to its relative fluorescence signal intensity at 60 min.

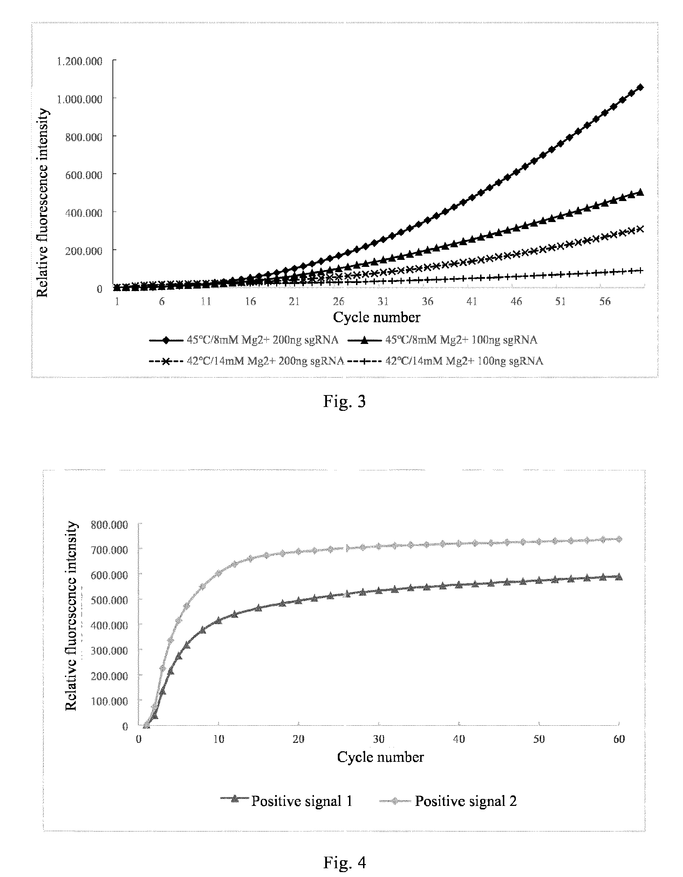

The obtained detection result is shown in Fig. 3, Table 7 and Table 8.

**Table 7. One-step CDetection reaction results under the high temperature reaction condition**

| Reaction results under the condition of 45°C/ 8mM Mg²⁺ | | | |
|---|---|---|---|
| Addition amount of sgRNA | *The height of the positive signal | **The height of the negative signal | ***Separation degree of positive and negative |
| 200ng sgRNA | 1,054,996 | 30,886 | 34.16 |
| 100ng sgRNA | 502,325 | 255,266 | 1.97 |
| 50ng sgRNA | 223,907 | 65,409 | 3.42 |
| 10ng sgRNA | 49,344 | 55,356 | 0.89 |
| 0ng sgRNA | 41,017 | 56,719 | 0.72 |

| | | | |
|---|---|---|---|
| *The height of the positive signal is the relative fluorescence signal intensity of FAM group with addition of 100 copies of viral genomic RNA after reacting at 45°C for 60 min, i.e., the result of subtracting fluorescence intensity of the baseline from the fluorescence intensity of FAM group at 60 min. **The height of the negative signal is the relative fluorescence signal intensity of the FAM group with addition of an equal volume of sample elution buffer into the reaction system instead of the viral genomic RNA after reacting at 45°C for 60 min. ***The separation degree of positive and negative is the ratio of the relative fluorescence signal intensity of the FAM group between the above positive reaction and negative reaction after reacting at 45°C for 60 min. | | | |

**Table 8. One-step CDetection reaction results under the low temperature reaction condition**

| Reaction results under the condition of 42°C/14mM Mg²⁺ | | | |
|---|---|---|---|
| Addition amount of sgRNA | *The height of the positive signal | **The height of the negative signal | ***Separation degree of positive and negative |
| 200ng sgRNA | 308,648 | 149,222 | 2.07 |
| 100ng sgRNA | 89,640 | 197,754 | 0.45 |
| 50ng sgRNA | 88,706 | 130,228 | 0.68 |
| 10ng sgRNA | 60,714 | 91,041 | 0.67 |
| 0ng sgRNA | 40,734 | 24,917 | 1.63 |

| | | | |
|---|---|---|---|
| *The height of the positive signal is the relative fluorescence signal intensity of FAM group with addition of 100 copies of viral genomic RNA for after reacting at 45°C 60 min, i.e., the result of subtracting fluorescence intensity of the baseline from the fluorescence intensity of FAM group at 60 min. **The height of the negative signal is the relative fluorescence signal intensity of the FAM group with addition of an equal volume of sample elution buffer into the reaction system instead of the viral genomic RNA after reacting at 42°C for 60 min. ***The separation degree of positive and negative is the ratio of the relative fluorescence signal intensity of the FAM group between the above positive reaction and negative reaction after reacting at 45°C for 60 min. | | | |

It can be seen from the above data that, when a higher CDetection reaction temperature is used, the specificity of the system tends to increase, which is reflected in the fact that the signal value of the negative reaction is sustained or decreased; at the same time, the positive signal is about 3.4 times higher than that of low temperature reaction in the case that the sgRNA is used in the normal dosage of the system (200ng).

### Example 4: Effect of reaction time on the reaction

### 1. Preparation of the reaction system according to the following table

**Table 9. Contents of addition order of components in the recombinase-aid isothermal amplification system**

| Component | Addition amount (µl) |
|---|---|
| Amplification buffer A | 12.5 |
| Magnesium acetate solution (280mM) | 2.5 |
| Transfer RNA (1000ng/µl) | 0.5 |
| Nuclease-free water | 31.125 |
| Suramin (800ng/µl) | 0.375 |
| Mixed solution of RAA forward and reverse primers (50µM) | 0.8 |
| RNA nuclease inhibitor (40U/ul) | 1.2 |
| Total (µl) | 50 |

| | |
|---|---|
| ¹ Transfer RNA was purchased from invitrogen with the catalog number AM7119. ² 50× TET buffer is a self-prepared buffer (100mM Tris pH8.0, 5mM EDTA, 0.5% Tween 20). The sequences of the forward and reverse primers are the same as those of Example 1. The sources of components such as amplification buffer A, magnesium acetate solution, and RNA nuclease inhibitor are the same as those in Example 1. ³ Evagreen dye (20x) was purchased from Biotium. | |

### 2. Loading samples for detection

After adding 1µl of pseudovirus nucleic acid sample (100 copies per microliter) to the above mixed solution, then placing in a real-time fluorescence quantitative PCR instrument to incubate for 1 min × 60 cycles at 45°C to observe the profile of the amplification curve.

The Evagreen dye was used to replace the FAM reporter group to characterize the amplification behavior of the target gene in the system, and the results are shown in Fig. 4.

As shown in the above figure, under the reaction condition of 45°C in the one-step reaction system, the reaction entered the linear amplification stage after 5-7 amplification cycles.

According to the results in the above figure, under the reaction condition of 45°C in the one-step reaction system, the reaction entered the linear amplification stage after 5-7 amplification cycles. At the same time, the Cas enzyme AaCas12b used in this method can maintain an optimal nuclease activity in a very wide temperature range (31-59°C). Therefore, under the consideration of not affecting the accumulation of RT-RAA reaction products, we kept the reaction conditions for the first 7 min at 45°C, and then the reaction temperature for 8-60 min was adjusted to 52°C. The key enzymes of RT-RAA were inactivated to exclude the effect of RT-RAA reaction components on the cleavage process, meanwhile the kinetics of the cleavage reaction was improved by increasing the temperature of the cleavage reaction, thereby accelerating the signal accumulation rate of the reporter group.

### Example 5: Effect of the additive on the reaction

Reverse transcription inhibition is relieved by using additive.

### 1. Pre-preparation of a sgRNA-Cas pre-preparation system (C mixture)

The sgRNA-Cas pre-preparation system was prepared according to the dosage of each component in Table 10 below, then placing on ice for use after the preparation was completed.

**Table 10. Contents and addition order of components in the sgRNA-Cas pre-preparation system**

| Component | Addition amount (µl) |
|---|---|
| AaCas12b protein (0.4mg/ml) | 0.6 |
| RNA nuclease inhibitor (40U/µl) | 1.2 |
| Single molecule guide RNA (200ng/µl) | 1.2 |
| Nuclease-free water | 2 |
| Total (µl) | 5 |

### 2. Pre-preparation of a recombinase-aid isothermal amplification system (A mixture)

The recombinase-aid isothermal amplification system was prepared according to the dosage of each component in Table 11 below, then placing at room temperature for use after the preparation was completed.

**Table 11. Contents and addition order of components in the recombinase-aid isothermal amplification system**

| Component | Addition amount (µl) |
|---|---|
| Nuclease-free water | 29.2-X1-X2 |
| Amplification buffer A | 12.5 |
| Magnesium acetate solution (280mM) | 2.5 |
| Transfer RNA (1000ng/µl) | X2 |
| Suramin (800ng/µl) | X1 |
| Mixed solution of RAA amplification forward and reverse primers (50µM) | 0.8 |
| Total (µl) | 45 |

Particularly, the sequence of the single-molecule guide RNA, and the sequences of the forward and reverse primers are the same as those in Example 1. The sources of components such as the amplification buffer A, magnesium acetate solution, the RNA nuclease inhibitor, the AaCas12b protein are the same as those in Example 1. The transfer RNA was purchased from Invitrogen with the catalog number AM7119.

### 3. Mix of the systems

45µl of A mixture, 1µl of viral nucleic acid sample (RNA sample was from 2019-nCoV RdRP pseudovirus and was extracted with Magen nucleic acid extraction kit, IVD5412, and after the extraction it was diluted to 50000 copies per microliter and 500 copies per microliter for use; DNA sample was a plasmid containing the 2019-nCovRdRP gene), and 5µl of C mixture were added into an empty tube. Particularly, the 2019-nCoV RdRP gene pseudovirus was purchased from Xiamen Zeesan Biotechnology Co., Ltd. with the catalog number JBD24; the 2019-nCovRdRP gene plasmid was purchased from GenScript Biotech Corp. (a chemical synthetic product without catalog number).

51µl of the above system was added into the enzyme dry powder (RT-RAA dry powder, purchased from ZC Bio-Sci&Tech Co., Ltd. with catalog number S0040ZC), then shaking for 8-10 seconds to mix well.

The above system was placed in a real-time fluorescence quantitative PCR instrument, the incubation program was: 45°C, 1 min × 60 cycles, collecting fluorescence once per minute. In this method, the FAM group was used as the reporter group, and the presence or absence of the target nucleic acid was judged according to its relative fluorescence signal intensity at 60 min.

The results are shown in Fig.5.

When 200ng of suramin and 500ng of tRNA were added at the same time, the separation degree data of negative and positive after reacting for 30 min obtained by using the detection method of the present application was shown in Table 12.

**Table 12. Results of the separation degree of negative and positive**

| Substrate | Separation degree of negative and positive* |
|---|---|
| 5E4 RNA | 7.50 |
| 5E4 dsDNA | 15.29 |
| 5E2 RNA | 3.28 |
| 5E2 dsDNA | 13.00 |

| | |
|---|---|
| *The separation degree of negative and positive refers to a ratio of the relative fluorescence intensity of the reporter group of the positive reaction / the relative fluorescence intensity of the reporter group of the negative reaction. | |

As shown in the above data, when suramin and tRNA were used as the additives in the mixed reaction system, and RNA was added as the substrate, there is a significant separation degree from the negative reaction, which relieves the inhibition effect of the one-step detection system of the present application on the reverse transcription reaction to a certain extent.

### Example 7: Effect of the dosage of the reporter probe on the detection result

### 1. Pre-preparation of a sgRNA-Cas pre-preparation system (C mixture)

The sgRNA-Cas pre-preparation system was prepared according to the dosage of each component in Table 13 below, then placing on ice for use after the preparation was completed.

**Table 13. Contents and addition order of components in the sgRNA-Cas pre-preparation system**

| Component | Addition amount (µl) |
|---|---|
| AaCas12b protein (0.4mg/ml) | 0.5 |
| RNA nuclease inhibitor (40U/µl) | 1.2 |
| Single molecule guide RNA (200ng/µl) | 1 |
| Magnesium acetate solution (280mM) | 1.43 |
| Nuclease-free water | 2.87 |
| Total (µl) | 7 |

### 2. Preparation of a recombinase-aid isothermal amplification system (A mixture)

The recombinase-aid isothermal amplification system was prepared according to the dosage of each component in Table 14 below, then placing at room temperature for use after the preparation was completed.

**Table 14. Contents and addition order of components in the recombinase-aid isothermal amplification system**

| Component | Addition amount (µl) |
|---|---|
| Nuclease-free water | 16.27-X |
| Amplification buffer A | 12.5 |
| Magnesium acetate solution (280mM) | 1.43 |
| Reporter probe (100uM) | X |
| Suramin (300ng/µl) | 2 |
| Mixed solution of RAA amplification forward and reverse primers (50µM) | 0.8 |
| Total (µl) | 33 |

Particularly, the sequence of the single-molecule guide RNA, the sequences of the forward and reverse primers and the sequence of the reporter probe are the same as those in Example 1. The sources of components such as the amplification buffer A, magnesium acetate solution, the RNA nuclease inhibitor, the AaCas12b protein are the same as those in Example 1. In this example, the final concentration experimental gradient of the probe was set as follows: 200nM, 400nM, 700nM, 1000nM, 1500nM and 2000nM, and the corresponding addition volumes (X) in the system are: 0.1µl, 0.4µl, 0.7µl, 1µl, 1.5µl, and 2µl respectively.

### 3. Mix of the systems

10µl of sample (a total of 2 samples were used in this experiment, one was water as the negative control, and the other was the positive sample of 8 copies/microlitre, wherein the positive sample was from 2019-nCoV RdRP Pseudovirus and was extracted with the Magen nucleic acid extraction kit, IVD5412, and after the extraction it was respectively diluted to 8 copies per microliter), and 33 µl of A mixture were respectively added into a tube containing the amplification reaction enzymedry powder. 7µl of C mixture was added into the corresponding tube cap, and then the tube cap was fastened. The above systems were fully shaken to mix well and centrifuged, and after the centrifugation it was shaken to mix well and centrifuged again. After the centrifugation, it was loaded on the detection instrument as soon as possible for fluorescence signal detection. Particularly, the 2019-nCoV RdRP gene pseudovirus was purchased from Xiamen Zeesan Biotechnology Co., Ltd. with the catalog number JBD249. The 2019-nCovRdRP gene plasmid was purchased from GenScript Biotech Corp. (a chemical synthetic product without catalog number).

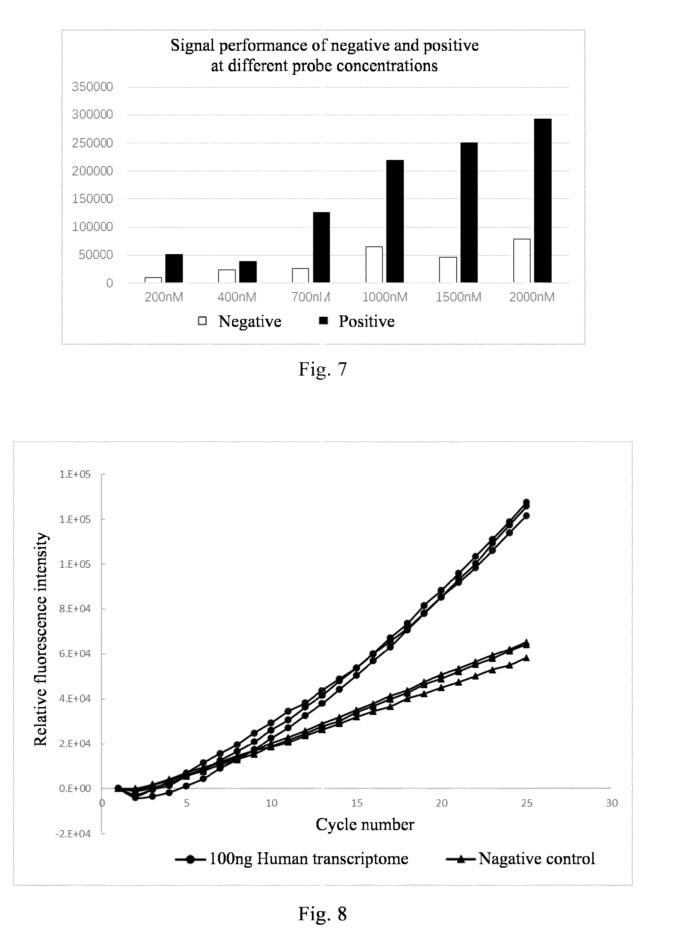

The above system was placed in a real-time fluorescence quantitative PCR instrument, and the incubation program was: 45°C for 7 min, collecting fluorescence once, (52°C for 1 min) × 22 cycles, collecting fluorescence once every minute, and 52°C for 1 min, collecting fluorescence once. In this method, the FAM group was used as the reporter group, and the presence or absence of the target nucleic acid was judged according to the relative fluorescence signal intensity at 30 min. The reaction results are shown in Fig. 7, and the relative fluorescence signal separation degrees of negative and positive are shown in Table 15.

**Table 15. Relative fluorescence signal separation degrees of negative and positive of different probe concentrations**

| Reporter probe concentration | Relative fluorescence signal separation degree of negative and positive |
|---|---|
| 200nM | 5.18 |
| 400nM | 1.69 |
| 700nM | 4.73 |
| 1000nM | 3.38 |
| 1500nM | 5.38 |
| 2000nM | 3.70 |

In this example, two biological replicates were performed for each probe concentration and each sample. The results shown in Fig. 7 are the average values of the two reactions. As shown in Fig. 7, increasing the concentration of the probe to be used can increase the signal intensity of the positive sample. As shown in Table 16, the results of this example show that the signal separation degrees of negative and positive at the reporter probe concentration of 200nM-2000nM are approximately equal to 2 or greater than 2, which meets the requirements for use. Therefore, it is recommended to use the reporter probe concentration of 200nM-2000nM.

### Test Example 1: 2019-nCoV RdRP gene detection

According to the effect analysis of the above factors, the following conditions were used to detect the target nucleic acid in the sample.

### 1. Pre-preparation of a sgRNA-Cas pre-preparation system (C mixture)

The sgRNA-Cas pre-preparation system was prepared according to the dosage of each component in Table 16 below, then placing on ice for use after the preparation was completed.

**Table 16. Contents and addition order of components in the sgRNA-Cas pre-preparation system**

| Component | Addition amount (µl) |
|---|---|
| AaCas12b protein (0.4mg/ml) | 0.6 |
| RNA nuclease inhibitor (40U/µl) | 1.2 |
| Single molecule guide RNA (200ng/µl) | 1 |
| Nuclease-free water | 2.2 |
| Total (µl) | 5 |

### 2. Preparation of a recombinase-aid isothermal amplification system (A mixture)

The recombinase-aid isothermal amplification system was prepared according to the dosage of each component in Table 17 below, then placing at room temperature for use after the preparation was completed.

**Table 17. Contents and addition order of components in the recombinase-aid isothermal amplification system**

| Component | Addition amount (µl) |
|---|---|
| Nuclease-free water | 17.5 |
| Amplification buffer A | 12.5 |
| Magnesium acetate solution (280mM) | 1.45 |
| Transfer RNA (1000ng/µl) | 1 |
| Suramin (800ng/µl) | 0.75 |
| Mixed solution of RAA amplification forward and reverse primers (50µM) | 0.8 |
| Reporter probe (62.5µM) | 1 |

Particularly, the sequence of the single-molecule guide RNA, the sequence of the reporter probe and the sequences of the forward and reverse primers are the same as those in Example 1. The sources of components such as the amplification buffer A, magnesium acetate solution, the RNA nuclease inhibitor, the AaCas12b protein are the same as those in Example 1. The transfer RNA was purchased from Invitrogen with the catalog number AM7119.

### 3. Mix of systems

10µl of the extracted pseudovirus nucleic acid (2019-nCoV RdRP gene pseudovirus, purchased from Xiamen Zeesan Biotechnology Co., Ltd with the catalog number JBD249, the nucleic acid was extracted with Magaen nucleic acid extraction kit, IVD5412, and after the extraction it was diluted to 10 copies or more per microliter for use) was taken to add into the ready-to-use reaction well. Then 35µl of A mixture of and 5µl of C mixture the recombinase-aid isothermal amplification system were respectively added into that well. 50µl of the above mixed solution was added into enzyme dry powder (RT-RAA dry powder, S0040ZC) to incubate at 45°C for 7min, and then incubate at 52°C for 1min× 23 cycles. In this method, the FAM group was used as the reporter group, and the presence or absence of the target nucleic acid was judged according to the relative fluorescence signal intensity at 30 min.

The obtained detection results are shown in Fig. 6.

Fig. 6 shows the CDetection reaction results of four viral genomic RNAs of 100 copies, and the CDetection reaction results of four negative (using an equal volume of sample elution buffer instead of viral nucleic acid sample).

On the ABI 7500 real-time fluorescence quantitative PCR instrument, the sample with the relative fluorescence signal intensity of the reporter group at 30min was judged whether there was a target nucleic acid, according to experience of the previous test data and CDetection reaction characteristics.

### Test Example 2: Test of human POP7 gene target

The gene target POP7 is often used as an internal reference for CRISPR-Cas related detection methods (James P. Broughton, 2020), and the test results of the second target POP7 gene (NM_005837.3) by the same method were provided in this Test Example, to verify the versatility of the method described in this application in the detection of different species and different targets.

### 1. Pre-preparation of a sgRNA-Cas pre-preparation system (C mixture)

The sgRNA-Cas pre-preparation system was prepared according to the dosage of each component in Table 18 below, then placing on an ice box or in a 4°C refrigerator for use after the preparation was completed.

**Table 18. Contents and addition order of components in the sgRNA-Cas pre-preparation system**

| Component | Addition amount (µl) |
|---|---|
| AaCas12b protein (0.4mg/ml) | 0.5 |
| RNA nuclease inhibitor (40U/µl) | 1.2 |
| Single molecule guide RNA (200ng/µl) | 1 |
| HEPES buffer (50x) | 1 |
| Nuclease-free water | 1.3 |
| Total (µl) | 7 |

| | |
|---|---|
| ¹ AaCas12b protein was purchased from Nanjing Vazyme Biotech Co.,Ltd. with the catalog number EN311-PE. ² RNA nuclease inhibitor was purchased from Promega, with the catalog number N2515. ³ The sequence of the single guide RNA is: 5'-GAGUCCUUUGGGCUUCCAGGCAACGGGUGUGCCAAUGGCCACUUUCCAGGU GGCAAAGCCCGUUGAACUUCAAGCGAAGUGGCACACUCAAUACUUGAGCACAC U-3' (SEQ ID NO: 4) | |

### 2. Preparation of a recombinase-aid isothermal amplification system (A mixture)

The recombinase-aid isothermal amplification system was prepared according to the dosage of each component in Table 19 below, then placing at room temperature for use after the preparation was completed.

**Table 19. Contents and addition order of components in recombinase-aid isothermal amplification system**

| Component | Addition amount (µl) |
|---|---|
| Nuclease-free water | 17.5 |
| Amplification buffer A^{l} | 12.5 |
| Magnesium acetate solution (280 mM)² | 1.45 |
| Tranfer RNA(1000 ng/µl) | 1 |
| Suramin (800 ng/µl) | 0.75 |
| Mixed solution of RAA amplification forward and reverse primers (50 µM)³ | 0.8 |
| Report probe (62.5 µM)⁴ | 1 |
| Total (µl) | 35 |

| | |
|---|---|
| ¹ Amplification buffer A is 4x RT-RAA buffer A purchased from sigma-aldrichwith the catalog number818892, and it was diluted to 20% for use. ² Magnesium acetate solution was purchased from sigma-aldrich with the catalog number 63052, and it was diluted to 280mM for use. ³ The sequence of the forward primer is: 5'-TGAGTACTGGACCTCGGACCAGAGCCATGTAAGAA-3' (SEQ ID NO:5), and the sequence of the reverse primer sequence is: 5'-GCCCACCAAGAGACAATTACCCCCACCCTCAATGC-3' (SEQ ID NO:6). ⁴ The sequence of the reporter probe is: 5'-FAM-TTTTTTT-BHQ1-3'. | |

### 3. Mix of the systems

10µl of POP7 nucleic acid sample (human 293T cell transcriptome, after extraction it was diluted to 10 ng/µl, 10 µl per reaction) was added into 35 µl of A mixture, followed by adding 5 µl of C mixture.

The above mixture was added to the enzyme dry powder (RT-RAA dry powder, purchased from Hangzhou ZC Bio-Sci&Tech Co., Ltd. with catalog number S0040ZC) to incubate at 45°C for 7 minutes, and then incubate at 52°C for 1 minute × 23 cycles, collecting fluorescence once per minute.

In this method, the FAM group was used as the reporter group, and the presence or absence of the target nucleic acid was judged according to the relative fluorescence signal intensity of the FAM group at 30 minutes. The results are shown in Fig. 8 and Table 20. The test results show that when 100ng of human transcriptome nucleic acid is added, the separation degree of fluorescence signals of positive sample and negative sample can reach a level of about 2 after reacting for 30 minutes.

**Table 20. Detection results of human transcriptome and Tween dilution group**

| Sample name | Initial fluorescence intensity | Fluorescence intensity at 30 minutes | Relative fluorescence intensity ¹ | Average relative fluorescence intensity | Separation degree of negative and positive ² |
|---|---|---|---|---|---|
| Human transcriptome | 594539.4 | 712172.1 | 117632.688 | 116929.5 | 1.965 |
| | 591233.2 | 705308.8 | 114075.563 | | |
| | 644658.4 | 763738.6 | 119080.188 | | |
| 0.01% Tween 20 | 549100.4 | 611171.5 | 62071.125 | 59505.2 | 1.000 |
| | 557125.3 | 618555.2 | 61429.875 | | |
| | 560187.4 | 615202.1 | 55014.688 | | |

| | | | | | |
|---|---|---|---|---|---|
| ¹ Relative fluorescence intensity = fluorescence intensity at 32 minutes - initial fluorescence intensity. ² Separation degree of negative and positive = the average relative fluorescence intensity of the sample / the average relative fluorescence intensity of 0.01 % Tween 20. | | | | | |

## Claims

1. A method for detecting a target nucleic acid in a sample, comprising the following steps:
reacting the sample with a mixed reaction system consisting of a sgRNA-Cas system and a recombinase-aid isothermal amplification system; and
detecting a detectable signal generated by the reaction after the reaction is completed;
wherein the sgRNA-Cas system comprises a Cas12b protein and a sgRNA targeting the target nucleic acid; the recombinase-aid isothermal amplification system comprises a primer, a single-stranded DNA reporter molecule that generates the detectable signal after being cleaved, suramin and tRNA;
wherein the target nucleic acid is RNA.

2. The method according to claim 1, wherein a concentration of the sgRNA in the mixed reaction system is 2-80ng/µl.

3. The method according to claim 1 or 2, wherein a concentration of the primer in the mixed reaction system is 300-1200nM.

4. The method according to any one of claims 1-3, wherein a concentration of the single-stranded DNA reporter molecule is 200-2000nM.

5. The method according to claim 4, wherein the concentration of the single-stranded DNA reporter molecule is 700-1500nM.

6. The method according to any one of claims 1-5, wherein the reaction is performed at a first temperature and inactivated at a second temperature, and the second temperature is greater than the first temperature.

7. The method according to claim 6, wherein the second temperature is greater than 47°C.

8. The method according to any one of claims 1-7, wherein the mixed reaction system further comprises a divalent cation, and a concentration of the divalent cation is 8-25 mM.

9. The method according to claim 8, wherein the divalent cation is one or more selected from the group consisting of: magnesium ion, calcium ion, manganese ion, cesium ion, nickel ion, iron ion, and cobalt ion.

10. The method according to any one of claims 1-9, wherein a concentration of the suramin in the mixed reaction system is 3-30ng/µl.

11. The method according to claim 10, wherein the concentration of the suramin in the mixed reaction system is 3-12ng/µl.

12. The method according to any one of claims 1-11, wherein a concentration of the tRNA in the mixed reaction system is 10-20ng/µl.

13. The method according to any one of claims 1-12, wherein the sample is selected from the group consisting of: whole blood, plasma, serum, cerebrospinal fluid, urine, feces, oral swab, nasopharyngeal swab, saliva, cell or tissue extract.

## Patentansprüche

1. Verfahren zum Nachweisen einer Zielnukleinsäure in einer Probe, umfassend die folgenden Schritte:
Reagieren der Probe mit einem gemischten Reaktionssystem, bestehend aus einem sgRNA-Cas-System und einem rekombinase-unterstützten isothermen Amplifikationssystem; und
Nachweisen eines durch die Reaktion erzeugten nachweisbaren Signals, nachdem die Reaktion abgeschlossen ist;
wobei das sgRNA-Cas-System ein Cas12b-Protein und eine sgRNA umfasst, die auf die Zielnukleinsäure abzielt; das rekombinase-unterstützte isotherme Amplifikationssystem einen Primer, ein einzelsträngiges DNA-Reportermolekül, das nach der Spaltung das nachweisbare Signal erzeugt, Suramin und tRNA umfasst;
wobei die Zielnukleinsäure RNA ist.

2. Verfahren nach Anspruch 1, wobei die Konzentration der sgRNA in dem gemischten Reaktionssystem 2-80 ng/µl beträgt.

3. Verfahren nach Anspruch 1 oder 2, wobei die Konzentration des Primers in dem gemischten Reaktionssystem 300-1200 nM beträgt.

4. Verfahren nach einem der Ansprüche 1 bis 3, wobei die Konzentration des einzelsträngigen DNA-Reportermoleküls 200-2000 nM beträgt.

5. Verfahren nach Anspruch 4, wobei die Konzentration des einzelsträngigen DNA-Reportermoleküls 700-1500nM beträgt.

6. Verfahren nach einem der Ansprüche 1 bis 5, wobei die Reaktion bei einer ersten Temperatur durchgeführt und bei einer zweiten Temperatur inaktiviert wird und die zweite Temperatur höher ist als die erste Temperatur.

7. Verfahren nach Anspruch 6, wobei die zweite Temperatur höher als 47 °C ist.

8. Verfahren nach einem der Ansprüche 1 bis 7, wobei das gemischte Reaktionssystem ferner ein zweiwertiges Kation umfasst und die Konzentration des zweiwertigen Kations 8-25 mM beträgt.

9. Verfahren nach Anspruch 8, wobei das zweiwertige Kation eines oder mehrere ausgewählt aus der Gruppe bestehend aus Magnesiumion, Calciumion, Manganion, Cäsiumion, Nickelion, Eisenion und Kobaltion ist.

10. Verfahren nach einem der Ansprüche 1 bis 9, wobei die Konzentration des Suramins in dem gemischten Reaktionssystem 3-30 ng/µl beträgt.

11. Verfahren nach Anspruch 10, wobei die Konzentration des Suramins in dem gemischten Reaktionssystem 3-12ng/µl beträgt.

12. Verfahren nach einem der Ansprüche 1 bis 11, wobei die Konzentration der tRNA in dem gemischten Reaktionssystem 10-20 ng/µl beträgt.

13. Verfahren nach einem der Ansprüche 1 bis 12, wobei die Probe ausgewählt ist aus der Gruppe bestehend aus: Vollblut, Plasma, Serum, Liquor cerebrospinalis, Urin, Fäkalien, Mundabstrich, Nasopharynxabstrich, Speichel, Zell- oder Gewebeextrakt.

## Revendications

1. Procédé de détection d'un acide nucléique cible dans un échantillon, comprenant les étapes suivantes :
la réaction de l'échantillon avec un système de réaction mixte consistant en un système sgRNA-Cas et un système d'amplification isotherme assistée par recombinase ; et
la détection d'un signal détectable généré par la réaction une fois la réaction terminée ;
dans lequel le système sgRNA-Cas comprend une protéine Cas12b et un sgRNA ciblant l'acide nucléique cible ; le système d'amplification isotherme assistée par recombinase comprend une amorce, une molécule d'ADN rapporteur simple brin qui génère le signal détectable après avoir été clivée, de la suramine et de l'ARNt ;
dans lequel l'acide nucléique cible est de l'ARN.

2. Procédé selon la revendication 1, dans lequel une concentration en sgRNA dans le système de réaction mixte est de 2-80 ng/µl.

3. Procédé selon la revendication 1 ou 2, dans lequel une concentration en amorce dans le système de réaction mixte est de 300-1 200 nM.

4. Procédé selon l'une quelconque des revendications 1-3, dans lequel une concentration en molécule d'ADN rapporteur simple brin est de 200-2 000 nM.

5. Procédé selon la revendication 4, dans lequel la concentration en molécule d'ADN rapporteur simple brin est de 700-1 500 nM.

6. Procédé selon l'une quelconque des revendications 1-5, dans lequel la réaction est effectuée à une première température et inactivée à une seconde température, et la seconde température est supérieure à la première température.

7. Procédé selon la revendication 6, dans lequel la seconde température est supérieure à 47 °C.

8. Procédé selon l'une quelconque des revendications 1-7, dans lequel le système de réaction mixte comprend en outre un cation divalent, et une concentration en cation divalent est de 8-25 mM.

9. Procédé selon la revendication 8, dans lequel le cation divalent est un ou plusieurs sélectionnés dans le groupe consistant en : un ion magnésium, un ion calcium, un ion manganèse, un ion césium, un ion nickel, un ion fer et un ion cobalt.

10. Procédé selon l'une quelconque des revendications 1-9, dans lequel une concentration en suramine dans le système de réaction mixte est de 3-30 ng/µl.

11. Procédé selon la revendication 10, dans lequel la concentration en suramine dans le système de réaction mixte est de 3-12 ng/µl.

12. Procédé selon l'une quelconque des revendications 1-11, dans lequel une concentration en ARNt dans le système de réaction mixte est de 10-20 ng/µl.

13. Procédé selon l'une quelconque des revendications 1-12, dans lequel l'échantillon est sélectionné dans le groupe consistant en : du sang total, du plasma, du sérum, du liquide céphalo-rachidien, de l'urine, des matières fécales, un frottis buccal, un échantillon prélevé par écouvillonnage du nasopharynx, de la salive, un extrait cellulaire ou tissulaire.
